# FASCICULE DE BREVET EUROPEEN

(11) **EP 0 845 271 B1**
(45) Date de publication et mention de la délivrance du brevet: **18.09.2002**
(21) Numéro de dépôt: 97402582.7
(22) Date de dépôt: 30.10.1997
(51) Int. Cl.: A61L 15/28

(54) **Produit de pansement à matrice d'alginate de calcium et son procédé de fabrication**
Verbandprodukt mit einer Kalziumalginatmatrix und Verfahren zur Herstellung
Dressing material having a calcium alginate matrix and method of making the same

(30) Priorité: 13.11.1996 FR 9613802
(43) Date de publication de la demande: 03.06.1998
(73) Titulaire: Société Précis, 92000 Nanterre (FR)
(72) Inventeur: Barikosky, Michel, 92000 Nanterre (FR); Maingault, Philippe, 49590 Fontevraud L'Abbaye (FR)
(74) Mandataire: Bloch, Gérard

(56) Documents cités:
- WO-A-92/22285
- WO-A-96/13282
- FR-A- 2 663 229
- FR-A- 2 671 486
- GB-A- 653 341
- GB-A- 1 328 088

## Description

La présente invention concerne tout d'abord un produit de pansement, ou pansement, pour plaies chroniques à liquide biologique, pour plaies humides, notamment profondes, telles que des cavités qui exudent comme les ulcères, les escarres ou autres plaies post-opératoires.

L'alginate de calcium est un polymère filable, comme tout alginate de métal de la famille des métaux multivalents, à l'exception du magnésium.

Si on considère un pansement comprenant une mèche de fibres d'alginate de calcium, qu'on applique dans une plaie avec perte de substance, ou de liquide biologique (sang ou exsudat), le pansement commence par absorber le liquide biologique qui suinte, ou qui exsude, les molécules d'eau du liquide s'intercalant entre les macromolécules de l'alginate. Une fois gonflé par absorption, le pansement est soumis à une gélification par échange ionique. Dans le cas de fibres d'alginate de calcium, elles cèdent des ions Ca²⁺ au liquide biologique qui leur cède des ions Na⁺. Au fur et à mesure que s'établit l'équilibre entre le calcium et le sodium, les fibres d'alginate perdent en partie leur structure cristalline. La gélification du pansement entraîne l'assèchement de la plaie et évite qu'il n'adhère aux tissus sousjacents.

Si on considère un pansement comprenant une matrice d'alginate de calcium associée à une couche d'alginate de sodium, comme enseigné par FR-A-2 663 229, dès la mise en contact de la couche d'alginate de sodium avec le fluide biologique, l'alginate de sodium, qui est un alginate de métal monovalent, passe en solution, qui inhibe la matrice d'alginate de calcium qui commence à perdre des ions Ca²⁺ et à prendre des ions Na⁺, avant que l'ensemble, par la poursuite de cet échange d'ions, ne soit réduit à l'état de gel.

On remarquera ici que la fonction de la couche d'alginate de sodium est de favoriser la gélification de la couche d'alginate de calcium sur une plaie peu exsudative.

Cependant, pour qu'une plaie chronique se cicatrice vraiment, il faut restaurer la physiologie de ses cellules, c'est-à-dire combler leur déficit en éléments minéraux métalliques essentiels, ionisés, qui, auparavant, c'est-à-dire avant l'apparition de la plaie, se trouvaient en faible quantité. Il s'agit ici de ce qu'on appelle les oligo-éléments et plus particulièrement les métaux multivalents (zinc, manganèse, cuivre, sélénium, etc.) à l'exception du magnésium.

Et bien la demanderesse a osé substituer à la couche d'alginate d'un métal monovalent, soluble, associée à la matrice d'alginate de calcium d'un pansement de l'art antérieur, de l'alginate d'un métal multivalent, à l'exception du magnésium, non soluble, à cause des ponts ioniques créés par le métal multivalent, mais dont les molécules vont quand même pouvoir être dissociées par les ions et cations monovalents du liquide biologique, pour proposer un pansement à pouvoir de restauration physiologique.

C'est ainsi que l'invention concerne un produit de pansement pour plaie chronique à liquide biologique, comprenant une matrice d'alginate de calcium associée à au moins un alginate de métal multivalent, à l'exception du magnésium.

Dès que le liquide biologique de la plaie vient au contact du pansement, les ions métalliques de l'un des deux alginates se libèrent, sans dissolution, avant que les ions de l'autre alginate n'en fassent de même, jusqu'à l'équilibre hydroélectrolytique entre les ions du liquide biologique, d'une part, et les ions des deux alginates, d'autre part, restaurant ainsi la physiologie des cellules de la plaie par apport d'ions du métal multivalent, c'est-à-dire de l'oligo-élément. Ainsi, le métabolisme en sommeil des cellules de fond de plaie peut être relancé.

Avantageusement, le métal multivalent de l'alginate associé à la matrice d'alginate de calcium est choisi dans le groupe comprenant le zinc, le manganèse, le cuivre, le sélénium.

Naturellement, à la matrice d'alginate de calcium peuvent être associés plusieurs alginates de plusieurs métaux multivalents, respectivement.

L'invention concerne également un procédé de fabrication d'un produit de pansement pour plaie chronique à liquide biologique, comprenant une matrice d'alginate de calcium associée à au moins un alginate de métal multivalent, à l'exception du magnésium, dans lequel on fabrique l'alginate de métal multivalent à partir d'alginate de sodium qu'on extrude dans une filière disposée dans un bain de coagulation d'une solution de chlorure du métal multivalent.

L'invention concerne encore un procédé de fabrication d'un produit de pansement pour plaie chronique à liquide biologique, comprenant une matrice d'alginate de calcium associée à au moins un alginate de métal multivalent, à l'exception du magnésium, dans lequel on associe l'alginate de métal multivalent à la matrice d'alginate de calcium par une technique textile.

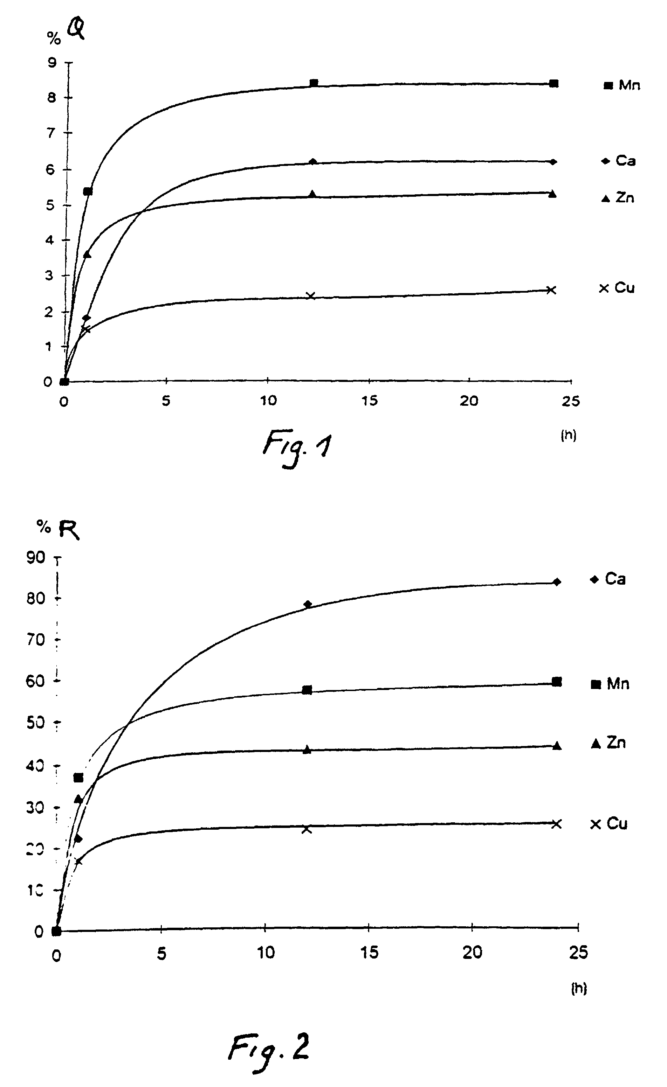

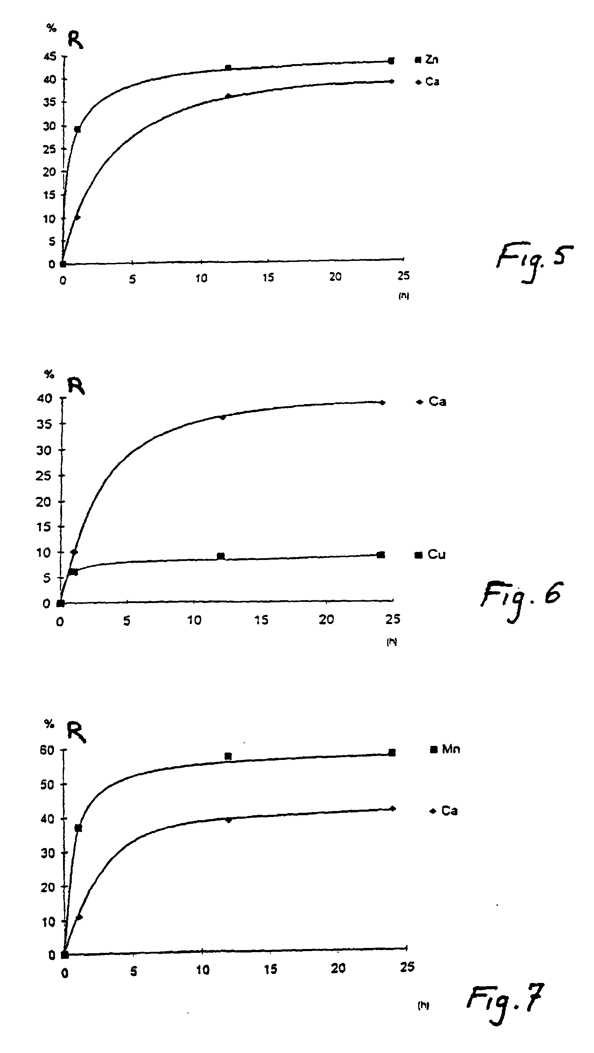

L'invention sera mieux comprise à l'aide de la description suivante du procédé de fabrication, chimique et mécanique, du pansement de l'invention et des figures 1 à 7 ci-jointes illustrant des résultats d'expériences rapportées ci-après.

### Au plan chimique

Pour l'alginate de calcium, on peut prendre des fibres d'alginate de calcium du commerce ou même le fabriquer.

Pour l'alginate de métal multivalent, magnésium excepté, de façon générale, on le fabrique de la manière suivante (des exemples précis seront présentés plus loin). On part d'alginate de sodium, hydrosoluble. On l'extrude à travers une filière qui est disposée dans un bain de coagulation d'une solution d'un sel soluble, par exemple un chlorure, du métal considéré. Par échange ionique entre le sodium et le métal, on obtient l'alginate du métal souhaité, non soluble, sous forme de fibres.

### Au plan mécanique

Dans le but d'obtenir des concentrations dites physiologiques, on préfère recourir à des techniques textiles qui permettent de maîtriser les quantités d'alginate fibrillaire tout en assurant l'homogénéité du pansement.

Les composés du pansement peuvent être, chacun, un non-tissé, un tissé, des flocks, de la poudre, des fils, un ruban de fibres, des fils de broderie, etc.

### 1) Deux non-tissés

1.1 On mélange intimement des fibres des deux alginates avant de les agglutiner en une nappe non tissée.
1.2 En variante, on replie sur lui-même un voile de non-tissé de chaque alginate en une nappe, on superpose les deux nappes et on soumet l'ensemble composite stratifié à un aiguilletage, avant d'obtenir une compresse.

### 2) Un non-tissé et un tissé (ou tricoté

On superpose une nappe non tissée d'un premier alginate et une nappe tissée (tricotée) du deuxième alginate. Après aiguilletage, on obtient une compresse. La nappe d'alginate de calcium peut être la nappe tissée ou non-tissée.

### 3) Un non-tissé et des flocks

A une nappe non-tissée d'un premier alginate, on fixe avec un agent de liaison, compatible avec les plaies, des flocks du deuxième alginate. Comme agent de liaison, on utilisera avantageusement un adhésif.

### 4) Un non-tissé et de la poudre

On procède comme ci-dessus avec des flocks.

### 5) Un non-tissé et des fils

On dispose les fils d'un premier alginate d'un côté ou de l'autre d'une nappe non tissée d'un deuxième alginate, ou entre deux nappes non tissées d'un ou deux autres alginates, et on soumet l'ensemble à un aiguilletage. On obtient alors une compresse.

### Les fils

On peut considérer trois techniques:
a) On peut utiliser des fils continus, à un ou plusieurs filaments, obtenus avec une filière;
b) on peut utiliser des fils obtenus à partir de torons de fibres étirées pour en détacher les fibres qu'on soumet ensuite
   - à l'action d'une retordeuse (technique du continu à filer) ou
   - à l'action d'une turbine pour entrelacer les fibres détachées et former des fils (technique open-end);
c) on peut utiliser des fils obtenus par la méthode DREFF bien connue de l'homme du métier, pour enrouler des fibres autour d'un ou plusieurs fils continus.

### 6) Un non-tissé et un ruban de fibres

On supperpose un ruban de fibres d'un premier alginate à une nappe non tissée d'un deuxième alginate et on soumet l'ensemble à un aiguilletage pour obtenir une compresse.

### 7) Un tissé et des flocks

On procède comme avec un non-tissé, à la seule différence que la nappe est tissée.

### 8) Un tissé et de la poudre

On procède comme avec un non-tissé, à la seule différence que la nappe est tissée.

### 9) Un non-tissé et des fils de broderie

On applique, sur une nappe non tissée d'un premier alginate, une broderie, issue d'une machine brodeuse, qu'on fixe à l'aide d'un agent de liaison, comme par exemple pour un non-tissé et des flocks.

### 10) Un tissé et des fils

A partir d'une nappe tissée d'un premier alginate, soit on y fixe, par exemple, une broderie d'un deuxième alginate, soit on y associe des fils du deuxième alginate par exemple selon le procédé Jacquard.

On a fait référence, jusqu'ici, à un pansement à seulement deux alginates. Naturellement, il est possible d'envisager toutes les compositions possibles d'alginate de calcium, d'une part, et d'un ou plusieurs alginates des métaux multivalents, magnésium excepté, d'autre part.

Vont maintenant être présentés deux exemples de fabrication de mélanges de fibres.

### EXEMPLE 1

Quatre types de fibres d'alginate ont été fabriqués, de même titre, ici 230 dTex, et dans les mêmes conditions de filature avec un paramétrage identique de l'équipement de filature au mouillé et
- une concentration de solution d'alginate de sodium de 6%,
- une concentration du sel dans le bain de coagulation de 1,5 M,
- des concentrations initiales de ions chélatés par l'alginate, exprimées en g d'ion pour 100 g de fibre sèche, qui sont les suivantes :
   - alginate de calcium 7,95 % Ca²⁺
   - alginate de zinc 11,87 % Zn²⁺
   - alginate de cuivre 10,24 % Cu²⁺
   - alginate de manganèse 14,74 % Mn²⁺

Des cinétiques de libération des ions métalliques ont été réalisées, par incubation des différentes fibres avec du soluté physiologique (NaCl 9 ‰), en mesurant la concentration de l'ion concerné dans la solution après 1h, 12h et 24h. Il y a des différences d'affinité des ions pour l'alginate, dans l'ordre suivant.

Cu²⁺ >>Zn²⁺ > Ca²⁺ > Mn²⁺

Ces différences sont illustrées par les courbes des figures 1 et 2 représentant la cinétique de libération des ions des alginates métalliques purs, c'est-à-dire l'évolution dans le temps, exprimé en heures h, des rapports Q en %, des quantités libérées, exprimées en g par 100 g de fibre (figure 1), et des rapports R, en %, des quantités libérées sur les quantités initiales (figure 2).

A 24 h, l'alginate de calcium a libéré 83 % de ses ions calcium, l'alginate de zinc 40 % de ses ions zinc, l'alginate de cuivre 25 % de ses ions cuivre, l'alginate de manganèse 59 % de ses ions manganèse. Ces taux correspondent à des maximums (les courbes sont pratiquement horizontales), et ce temps de 24 h correspond au temps moyen de séjour d'un pansement sur une plaie.

Après coupe des fibres en flocks, le mélange suivant a été fabriqué :
alginate de calcium 76 %
alginate de zinc 8 %
alginate de cuivre 8 %
alginate de manganèse 8 %.

Les cinétiques de libération ont été réalisées comme précédemment, à chaque temps ayant été mesurée la concentration des quatre espèces ioniques présentes simultanément.

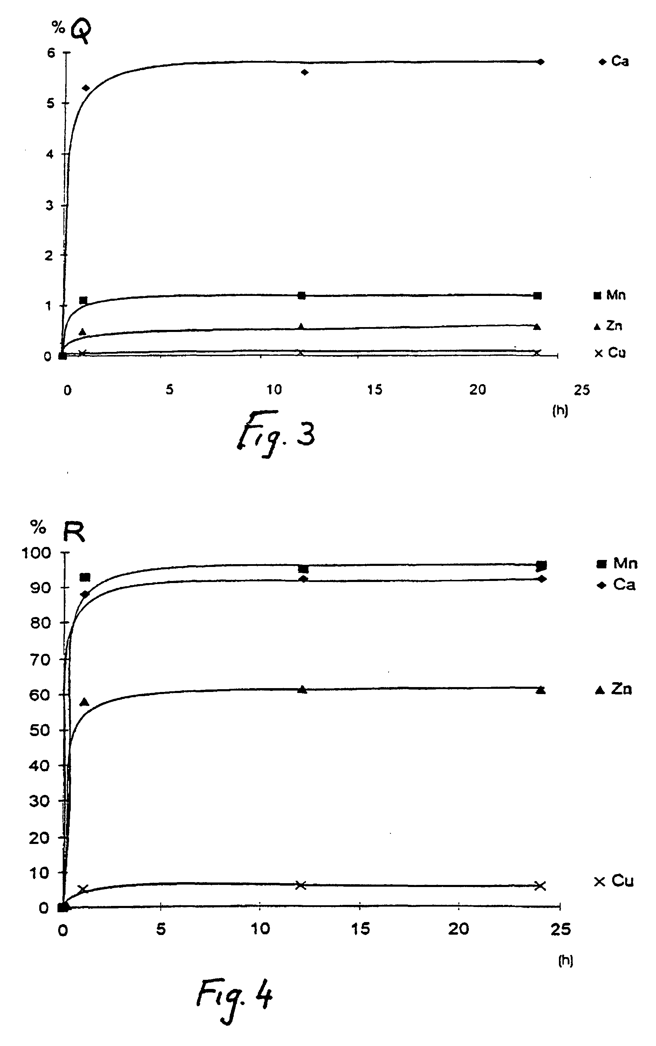

Les résultats sont illustrés par les courbes des figures 3 et 4 représentant la cinétique de libération des ions d'alginates métalliques associés (calcium, zinc, manganèse, cuivre) et plus particulièrement l'évolution des rapports Q (figure 3) et R (figure 4) tels que définis ci-dessus. Ces résultats démontrent que les concentrations d'ions libérés varient par rapport aux résultats des figures 1 et 2. Il existe des mécanismes de compétition (agonisme et antagonisme).

A 24 h, le mélange a libéré 92 % de ses ions calcium, 60 % de ses ions zinc, 6 % de ses ions cuivre et 96 % de ses ions manganèse.

La maîtrise des concentrations des oligo-éléments dans la plaie dépend donc :
- des espèces initiales dans le mélange,
- des quantités initiales dans le mélange,
- de la maîtrise du mélange des fibres.

### EXEMPLE 2

Des fibres d'alginate des mêmes types et dans les mêmes conditions que dans l'exemple 1 ont été fabriquées.

Trois mélanges A, B, C ont été réalisés, par broderie d'un fil d'alginate métallique sur un non-tissé d'alginate de calcium

| | A | B | C |
|---|---|---|---|
| Alginate de calcium | 99,96 % | 99,96 % | 99,97 % |
| alginate de zinc | 0,04 % | | |
| alginate de cuivre | | 0,04 % | |
| alginate de manganèse | | | 0,003 % |

Les cinétiques de libération ont été réalisées comme dans l'exemple 1. Les résultats sont illustrés par les courbes des figures 5 - 7 représentant la cinétique de libération des ions d'alginates métalliques associés, c'est-à-dire l'évolution des rapports R, tels que définis ci-dessus, pour les trois mélanges A (figure 5), B (figure 6) et C (figure 7).

Les résultats obtenus montrent que l'on peut maîtriser des concentrations très faibles (physiologiques) en oligo-éléments et que les cinétiques avec deux espèces ioniques métalliques diffèrent de celles obtenues précédemment puisque les mécanismes de compétition sont plus simples.

On notera également que, outre la nature de l'oligo-élément, sa concentration initiale influence les cinétiques : on met ici en évidence que l'équilibre ionique repose sur les coefficients d'affinité des différents ions pour l'alginate (exemple 1) et sur les concentrations des différentes espèces.

## Revendications

1. Produit de pansement pour plaie chronique à liquide biologique, comprenant une matrice d'alginate de calcium associée à au moins un alginate de métal multivalent, à l'exception du magnésium.

2. Produit de pansement selon la revendication 1, dans lequel plusieurs alginates de plusieurs métaux multivalents sont associés à la matrice d'alginate de calcium.

3. Produit de pansement selon l'une des revendications 1 et 2, dans lequel le métal multivalent de l'alginate associé à la matrice d'alginate de calcium est choisi dans le groupe comprenant le zinc, le manganèse, le cuivre, le sélénium.

4. Procédé de fabrication d'un produit de pansement pour plaie chronique à liquide biologique, comprenant une matrice d'alginate de calcium associée à au moins un alginate de métal multivalent, à l'exception du magnésium, dans lequel on fabrique l'alginate de métal multivalent à partir d'alginate de sodium qu'on extrude dans une filière disposée dans un bain de coagulation d'une solution d'un sel soluble du métal multivalent.

5. Procédé de fabrication d'un produit de pansement pour plaie chronique à liquide biologique, comprenant une matrice d'alginate de calcium associée à au moins un alginate de métal multivalent, à l'exception du magnésium, dans lequel on associe l'alginate de métal multivalent à la matrice d'alginate de calcium par une technique textile.

6. Procédé de fabrication selon la revendication 5, dans lequel, pour associer la matrice d'alginate de calcium à l'alginate de métal multivalent, on part d'une nappe non tissée d'alginate de calcium et d'alginate de métal multivalent se présentant sous l'une des formes de l'ensemble comprenant une nappe non tissée, une nappe tissée, des flocks, de la poudre, des fils, de la broderie, un ruban de fibres.

7. Procédé de fabrication selon la revendication 5, dans lequel, pour associer la matrice d'alginate de calcium à l'alginate de métal multivalent, on part d'une nappe tissée d'alginate de calcium et d'alginate de métal multivalent se présentant sous l'une des formes de l'ensemble comprenant des flocks, de la poudre, des fils.

## Patentansprüche

1. Verbandmaterial für eine chronische Wunde mit biologischer Flüssigkeit, eine Calciumalginatmatrix umfassend, die mit mindestens einem Alginat eines multivalenten Metalls, mit Ausnahme von Magnesium, assoziiert ist.

2. Verbandmaterial nach Anspruch 1, **dadurch gekennzeichnet, dass** mehrere Alginate von mehreren multivalenten Metallen mit der Calciumalginatmatrrix assoziiert sind.

3. Verbandmaterial nach einem der Ansprüche 1 und 2, **dadurch gekennzeichnet, dass** das multivalente Metall des mit der Calciumalginatmatrix assoziierten Alginats aus der Gruppe ausgewählt ist, die Zink, Mangan, Kupfer und Selen umfasst.

4. Verfahren zur Herstellung eines Verbandmaterials für eine chronische Wunde mit biologischer Flüssigkeit, wobei das Verbandmaterial eine Caiciumalginatmatrix umfaßt, die mit mindestens einem Alginat eines multivalenten Metalls, mit Ausnahme von Magnesium, assoziiert ist, bei dem das Alginat eines multivalenten Metalls aus Natriumalginat hergestellt wird, das in einer Düse extrudiert wird, die in einem Gerinnungsbad einer Lösung eines löslichen Salzes des multivalenten Metalls angeordnet ist.

5. Verfahren zur Herstellung eines Verbandmaterials für eine chronische Wunde mit biologischer Flüssigkeit, wobei das Verbandmaterial eine Calciumalginatmatrix umfaßt, die mit mindestens einem Alginat eines multivalenten Metalls, mit Ausnahme von Magnesium, assoziiert ist, bei dem das Alginat des multivalenten Metalls mit der Calciumalginatmatrix mittels einer Textiltechnik assoziiert wird.

6. Herstellungsverfahren nach Anspruch 5, **dadurch gekennzeichnet, dass** zur Assoziierung der Calciumalginatmatrix mit dem Alginat des multivalenten Metalls von einem nicht gewebten Vlies aus Calciumalginat und Alginat eines multivalenten Metalls ausgegangen wird, das in einer der Formen aus der Gruppe vorliegt, die nichtgewebtes Vlies, gewebtes Vlies, Flocken, Pulver, Fäden, Stickmaterial, Faserband umfasst.

7. Herstellungsverfahren nach Anspruch 5, **dadurch gekennzeichnet, dass** zur Assoziierung der Calciumalginatmatrix mit dem Alginat des multivalenten Metalls von einem gewebten Vlies aus Calciumalginat und Alginat eines multivalenten Metalls ausgegangen wird, das in einer der Formen aus der Gruppe vorliegt, die Flocken, Pulver, Fäden umfaßt.

## Claims

1. Dressing product for a chronic wound with biological fluid, comprising a calcium alginate matrix associated with at least one alginate of a multivalent metal, with the exception of magnesium.

2. Dressing product according to claim 1, wherein a plurality of alginates of a plurality of multivalent metals are associated with the calcium alginate matrix.

3. Dressing product according to one of claims 1 and 2, wherein the multivalent metal of the alginate associated with the calcium alginate matrix is chosen from the group comprising zinc, manganese, copper, selenium.

4. Method of manufacturing a dressing product for a chronic wound with biological fluid, comprising a calcium alginate matrix associated with at least one alginate of a multivalent metal, with the exception of magnesium, wherein the multivalent metal alginate is manufactured from sodium alginate which is extruded in a die disposed in a coagulation bath of a solution of a soluble salt of the multivalent metal.

5. Method of manufacturing a dressing product for a chronic wound with biological fluid, comprising a calcium alginate matrix associated with at least one alginate of a multivalent metal, with the exception of magnesium, wherein the multivalent metal alginate is associated with the calcium alginate matrix by means of a textile technique.

6. Manufacturing method according to claim 5, wherein, in order to associate the calcium alginate matrix with the multivalent metal alginate, one starts from a non-woven sheet of calcium alginate and of multivalent metal alginate presented in one of the forms of the assembly comprising a non-woven sheet, a woven sheet, flocks, powder, threads, embroidery, a band of fibres.

7. Manufacturing method according to claim 5, wherein, in order to associate the calcium alginate matrix with the multivalent metal alginate, one starts from a woven sheet of calcium alginate and of multivalent metal alginate presented in one of the forms of the assembly comprising flocks, powder, threads.
